# FASCICULE DE BREVET EUROPEEN

(11) **EP 1 390 367 B1**
(45) Date de publication et mention de la délivrance du brevet: **09.07.2008**
(21) Numéro de dépôt: 02738249.8
(22) Date de dépôt: 16.05.2002
(51) Int. Cl.: C07D 471/14, A61K 31/445

(54) **DERIVES DE DIHYDROIMIDAZO¬5,1-A -BETA-CARBOLINE, LEUR PROCEDE DE PREPARATION ET LEUR APPLICATION A TITRE DE MEDICAMENT**
DIHYDROIMIDAZO¬5,1-A -BETA-CARBOLIN-DERIVATE, VERFAHREN ZU IHRER HERSTELLUNG UND IHRE ANWENDUNG ALS ARZNEIMITTEL
DIHYDROIMIDAZO 5,1-A -$G(b)-CARBOLINE DERIVATIVES, METHOD FOR PREPARING SAME AND USE THEREOF AS MEDICINE

(30) Priorité: 16.05.2001 FR 0106444
(43) Date de publication de la demande: 25.02.2004
(73) Titulaire: MACEF, 86440 Migne-Auxances (FR)
(72) Inventeur: FOURTILLAN, Jean-Bernard, 33000 Bordeaux (FR); FOURTILLAN, Marianne, 33000 Bordeaux (FR); KARAM, Omar, 86280 Saint Benoit (FR); ZUNINO, Fabien, F-86180 Buxerolles (FR); JACQUESY, Jean-Claude, F-86180 Buxerolles (FR); TAFANI, Jean-Pierre, F-94700 Maisons Alfort (FR)
(74) Mandataire: Warcoin, Jacques
(86) Numéro de dépôt international: PCT/FR2002/001653
(87) Numéro de publication internationale: WO 2002/092598

(56) Documents cités:
- WO-A-95/24191
- WO-A-96/08490
- WO-A-99/47521
- DATABASE CA [en ligne] CHEMICAL ABSTRACTS SERVICE, COLUMBUS, OHIO, US; KANAOKA, YUICHI ET AL: "Amino acids and peptides. II. Cyclodehydration of some tryptophan-dipeptides and their derivatives with a polyphosphate ester" retrieved from STN Database accession no. 68:78590 XP002185124 & TETRAHEDRON (1968), 24(6), 2591-4, cité dans la demande

## Description

La demande brevet WO 99/47521 décrit des dérivés hypnotiques de béta-carboline, leur procédé de préparation et leur utilisation en tant que produit médicinal. Les composés décrits dans ce document sont des composés comportant un cycle fusionné au noyau tricyclique beta-carboline, ce cycle étant une pyridine hydrogénée et non une imidazole.

La demande de brevet WO 96/08490 décrit de nouveaux dérivés de béta-carboline et d'analogues, agoniste de la mélatonine, leur procédé de préparation et leur utilisation à titre de médicament. Toutefois, les composés décrits ne contiennent pas de cycle fusionné au noyau tricyclique béta-carboline et en particulier d'imidazole.

La demande de brevet WO 95/24191 décrit des tétrahydro-béta-carboline substitués en position 8, ayant une activité sur le système nerveux central. Toutefois, ce document ne décrit pas de composés ayant un cycle fusionné au noyau tricyclique béta-carboline et en particulier d'imidazole.

La présente invention concerne de nouveaux dérivés de dihydroimidazo[5,1-a]-β-carboline de formule générale (I) 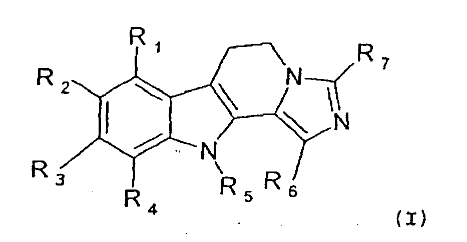 dans laquelle : R₁, R₃ et R₄ représentent un atome d'hydrogène, R₂ représente un atome d'hydrogène, d'halogène, un groupement alkyle (C₁-C₆) linéaire ou ramifié, hydroxy, alkoxy (C₁-C₆) linéaire ou ramifié, trihalogénoalkyle (C₁-C₆) linéaire ou ramifié ou trihalogénoalkoxy (C₁-C₆) linéaire ou ramifié,

R₅ représente un atome d'hydrogène ou un groupement alkyle (C₁-C₆) linéaire ou ramifié,

R₆ et R₇ identiques ou différents, indépendamment l'un de l'autre, représentent un atome d'hydrogène, un groupe alkyle (C₁-C₆) linéaire ou ramifié, ou un phényle
ainsi que leurs sels d'addition à un acide pharmaceutiquement acceptable.

Dans la présente description, par aryle, on comprend un groupement phényle, naphtyle, tétrahydronaphtyle, dihydronaphtyle, indényle ou indanyle, chacun de ces groupements étant éventuellement substitué de façon identique ou différente, par un ou plusieurs atomes d'halogène, groupements hydroxy, cyano, nitro, alkyle (C₁-C₆) linéaire ou ramifié, alkoxy (C₁-C₆) linéaire ou ramifié, amino, alkyle (C₁-C₆) amino linéaire ou ramifié, dialkyle (C₁-C₆) amino linéaire ou ramifié, aryloxy, arylalkoxy (C₁-C₆) linéaire ou ramifié, trihalogénoalkyle (C₁-C₆) linéaire ou ramifié, acyle (C₁-C₆) linéaire ou ramifié, alkoxycarbonyle (C₁-C₆) linéaire ou ramifié, alkylaminocarbonyle (C₁-C₆) linéaire ou ramifié, ou oxo.

Les substituants R₂ préférés selon l'invention sont l'atome d'hydrogène, un atome d'halogène (fluor, chlore ou brome), un alkyle (C₁-C₆) linéaire ou ramifié), un hydroxy, un alkoxy (C₁-C₆) linéaire ou ramifié)

Les composés préférés selon l'invention sont :
3,11-diméthyl-5,6-dihydroimidazo[5,1-a]-β-carboline méthane sulfonate
8-méthoxy-3-méthyl-5,6-dihydroimidazo[5,1-a]-β-carboline méthane sulfonate
8-méthoxy-5,6-dihydroimidazo[5,1-a]-β-carboline
8-méthoxy-3-méthyl-1-phényl-5,6-dihydroimidazo[5,1-a]-β-carboline
1-éthyl-8-méthoxy-3-méthyl-5,6-dihydroimidazo[5,1-a]-β-carboline
8-méthoxy-3-isopropyl-5,6-dihydroimidazo[5,1-a]-β-carboline
8-méthoxy-3-propyl-5,6-dihydroimidazo[5,1-a]-β-carboline
8-méthoxy-11-méthyl-3-propyl-5,6-dihydroimidazo[5,1-a]-β-carboline méthane sulfonate
8-méthoxy-3,11-diméthyl-5,6-dihydroimidazo[5,1-a]-β-carboline méthane sulfonate
8-hydroxy-3,11-diméthyl-5,6-dihydroimidazo[5,1-a]-β-carboline méthane sulfonate
8-hydroxy-3-méthyl-5,6-dihydroimidazo[5,1-a]-β-carboline méthane sulfonate
3-méthyl-5,6-dihydroimidazo[5,1-a]-β-carboline méthane sulfonate
8-chloro-3-méthyl-5,6-dihydroimidazo[5,1-a]-β-carboline méthane sulfonate
8-méthoxy-3-phényl-5,6-dihydroimidazo[5,1-a]-β-carboline
11-éthyl-3-méthyl-5,6-dihydroimidazo[5,1-a]-β-carboline méthane sulfonate
8-chloro-3,11-diméthyl-5,6-dihydroimidazo[5,1-a]-β-carboline méthane sulfonate
8-chloro-3-méthyl-11-ethyl-5,6-dihydroimidazo[5,1-a]-β-carboline méthane sulfonate
3,8-diméthyl-5,6-dihydroimidazo[5,1-a]-β-carboline méthane sulfonate
3,8,11-triméthyl-5,6-dihydroimidazo[5,1-a]-β-carboline méthane sulfonate
11-éthyle-3,8-diméthyl-5,6-dihydroimidazo[5,1-a]-β-carboline méthane sulfonate
8-fluoro-3-méthyl-5,6-dihydroimidazo[5,1-a]-β-carboline méthane sulfonate
8-bromo-3-méthyl-5,6-dihydroimidazo[5,1-a]-β-carboline méthane sulfonate
8-fluoro-3,11 diméthyl-5,6-dihydroimidazo[5,1-a]-β-carboline méthane sulfonate
8-fluoro-11-éthyl-3-méthyl-5,6-dihydroimidazo[5,1-a]-β-carboline méthane sulfonate
8-bromo-11-éthyl-3-méthyl-5,6-dihydroimidazo[5,1-a]-β-carboline méthane sulfonate

L'invention s'étend également au procédé de préparation des composés de formule (I) caractérisé en ce qu'on utilise comme produit de départ un composé de formule (II) 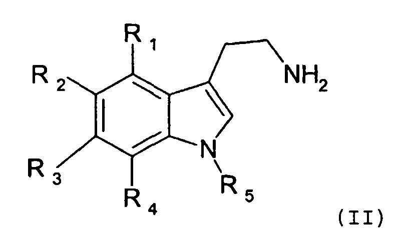 dans lequel R₁, R₂, R₃, R₄ et R₅ ont la même signification que dans la formule (I), composé de formule (II) qui est mis à réagir, selon des conditions de synthèse de type couplage peptidique, avec un composé de formule (III) : 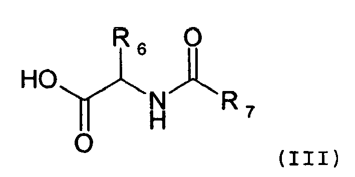 dans laquelle R₆ et R₇ sont tels que définis dans la formule (I), pour conduire au composé de formule (IV) : 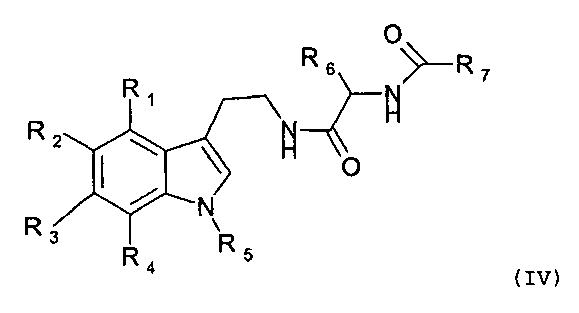 dans lequel R₁, R₂, R₃, R₄, R₅, R₆ et R₇ sont tels que définis précédemment, composé de formule (IV) que l'on traite en présence d'oxychlorure de phosphore dans un solvant tel que le toluène, pour conduire aux composés de formule (I). 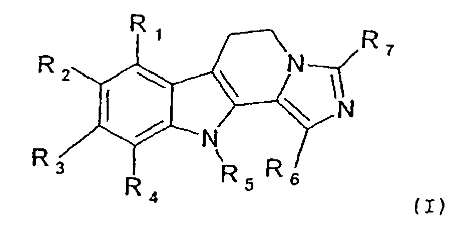 dans lequel R₁, R₂, R₃, R₄, R₅, R₆ et R₇ sont tels que définis précédemment, les composés de formule générale (I) formant l'ensemble des composés de l'invention, que l'on transforme, le cas échéant, en leurs sels d'addition à un acide pharmaceutiquement acceptable.

Les composés de formule (II) et (III) sont soit des composés commerciaux soit obtenus selon des méthodes connues de la synthèse organique.

La présente invention a également pour objet les compositions pharmaceutiques renfermant comme principe actif au moins un composé de formule (I), ses sels d'addition à un acide pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveinenuse, intramusculaire ou sous-cutanée), per ou transcutanée, nasale, rectale, perlinguale, oculaire ou respiratoire, et notamment les comprimés simples ou dragéifiés, les comprimés sublinguaux, les sachets, les gélules, les tablettes, les suppositoires, les crèmes, les pommades, les gels dermiques, les préparations injectables ou buvables, les aérosols, les gouttes oculaires ou nasales.

Des dérivés de dihydroimidazo[5,1-a]-β-carboline ont été décrits dans l'état de la technique comme intermédiaires de synthèse,
R₁=R₂=R₃=R₄=R₅=R₆=H et R₇=CH₃ ; Kanaoke, Y; Sato, E; Yonemitsu, O, Tetrahedron, 1968, 24, 2591-2594

R₁=R₂=R₃=R₄=R₅=R₆=H et R₇=Ph , Elliott, J.Org.Chem, 1962, 3302-3305.

Bien entendu ces dérivés, ne sont décrits que comme intermédiaires de synthèse et n'ont pas d'activité thérapeutique connue. Ils ne font partie de la présente invention qu'à titre de médicament en particulier comme hypnotique.

Les produits de départ et/ou les réactifs utilisés sont des produits connus ou préparés selon des modes opératoires connus.

Les structures des composés décrits dans les exemples et les stades de synthèse ont été déterminés selon les techniques spectrophotométriques usuelles (infrarouge, RMN, spectroscopie de masse).

Pour illustrer l'objet de la présente invention on indiquera ci-après quelques exemples de dérivés de formule générale I, pour lesquels R₁=R₃=R₄=H

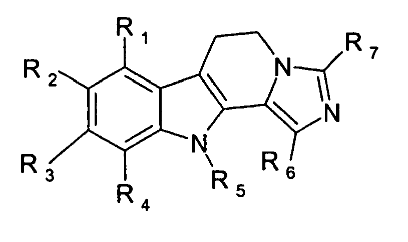

**Tableau I**

| **EXEMPLE** | **R₂** | **R₅** | **R₆** | **R₇** |
|---|---|---|---|---|
| **Exemple 1** | H | CH₃ | H | CH₃ |
| **Exemple 2** | CH₃O | H | H | CH₃ |
| **Exemple 3** | CH₃O | H | H | H |
| **Exemple 4** | CH₃O | H | Ph | CH₃ |
| **Exemple 5** | CH₃O | H | CH₃CH₂ | CH₃ |
| **Exemple 6** | CH₃O | H | H | CH(CH₃)₂ |
| **Exemple 7** | CH₃O | H | H | (CH₂)₂CH₃ |
| **Exemple 8** | CH₃O | CH₃ | H | (CH₂)₂CH₃ |
| **Exemple 9** | CH₃O | CH₃ | H | CH₃ |
| **Exemple 10** | OH | CH₃ | H | CH₃ |
| **Exemple 11** | OH | H | H | CH₃ |
| **Exemple 12** | H | H | H | CH₃ |
| **Exemple 13** | Cl | H | H | CH₃ |
| **Exemple 14** | CH₃O | H | H | Ph |
| **Exemple 15** | H | CH₃CH₂ | H | CH₃ |
| **Exemple 16** | Cl | CH₃ | H | CH₃ |
| **Exemple 17** | Cl | CH₃CH₂ | H | CH₃ |
| **Exemple 18** | CH₃ | H | H | CH₃ |
| **Exemple 19** | CH₃ | CH₃ | H | CH₃ |
| **Exemple 20** | CH₃ | CH₃CH₂ | H | CH₃ |
| **Exemple 21** | F | H | H | CH₃ |
| **Exemple 22** | Br | H | H | CH₃ |
| **Exemple 23** | F | CH₃ | H | CH₃ |
| **Exemple 24** | F | CH₃CH₂ | H | CH₃ |
| **Exemple 25** | Br | CH₃CH₂ | H | CH₃ |

### Exemple 1 : 3,11-diméthyl-5,6-dihydroimidazo[5,1-a]-β-carboline méthane sulfonate

### Mode opératoire A : 2-Acétylamino-N-[2-(1H-indol-3-yl)-éthyl]acétamide

A un mélange de tryptamine (4,32g, 27 mmol) et de N-acétylglycine (3,3g, 28 mmol) dans du DMF (100 ml) refroidie à 0°C,on ajoute successivement le diphénylphosphorylazide (5,8 ml, 27,5 ml) et la triéthylamine (3,85 ml, 27,5 ml). L'ensemble est agité sous azote à température ordinaire pendant 12 h, le solvant est éliminé sous pression réduite. Le résidu obtenu est flash chromatographié sur gel de silice pour conduire au produit attendu (m=5,5 g, 21 mmol) soit un rendement de 78%.

### Mode opératoire B : 2-Acétylamino-N-[2-(1-méthyl-1H-indol-3-yl)-éthyl]acétamide

A l'amide (2g, 8,23 mmol) obtenu avec le *mode opératoire* A dans du DMF (20ml) sont additionnés du NaH à 60% dans l'huile (0,35g, 8,75 mmol) et l'halogénure d'alkyle (CH₃I -0,55 ml, 8,83 mmol). On agite pendant 12 heures à température ordinaire avant d'éliminer le solvant sous pression réduite. On obtient alors après flash chromatographie sur gel de silice le produit attendu (1,02g, 3,96 mmol) soit un rendement de 48%.

### Mode opératoire C : 3,11-diméthyl-5,6-dihydroimidazo[5,1 a]-β-carboline méthane sulfonate

L'amide (1,02g, 3,96 mmol) *mode opératoire* B est porté à reflux dans du toluène (V=50m1), on ajoute pendant 30 mn du POCl₃ (10ml) dans du toluène (15 ml). Le milieu réactionnel est concentré sous pression réduite et le résidu est repris avec de l'éthanol (5ml) puis est ajoutée NaOH (20%, 50ml). On laisse sous agitation pendant 30 mn, le solide formé est récupéré par filtration. Le produit est purifié par flash chromatographie sur gel de silice, on obtient le produit attendu (280 mg, 1,26mmol , Rdt=32%).

La *3,11-diméthyl-5,6-dihydroimidazo[5,1-a]-β-carboline* méthane *sulfonate* dissout dans de l'éthanol, on ajoute l'acide méthane sulfonique (léquivalent), on obtient par précipitation le mésylate correspondant.
*RMN ¹H (300 MHz, CDCl₃)* 2,72 (s, 3H); 2,78 (s, 3H); 3,23 (t, J=6,9 Hz, 2H), 3,71 (s, 3H); 4,22 (t, J=6,9 Hz, 2H); 7,11 (m, 2H); 7,24 (s, 2H), 7,48 (m, 2H)
*SM (m*/*z)* 237 (100); 221; 195; 181.

### Exemple 2 : 8-méthoxy-3-méthyl-5,6-dihydroimidazo[5,1-a]-β-carboline méthane sulfonate

On procède comme dans l'exemple 1, en utilisant comme substrat lors du *mode opératoire A* la 5-méthoxytryptamine et la N-acétylglycine, l'amide obtenu est directement engagé dans la réaction de cyclisation *mode opératoire* C.

*RMN ¹H (300 MHz, CDCl₃)* 2,40 (s, 3H); 2,80 (s, 3H); 3,00 (t, J=6,6 Hz, 2H); 3,80 (s, 3H); 3,96 (t, J=6,6 Hz, 2H); 6,78 (m, 2H); 7,20 (d, J=8,8 Hz, 1H); 7,50 (s,1H).

### Exemple 3 : 8-méthoxy-5,6-dihydroimidazo[5,1-a]-β-carboline

On procède comme dans l'exemple 1, en utilisant comme substrat lors du *mode opératoire A* la 5-méthoxytryptamine et la N-formylglycine, l'amide obtenu est directement engagé dans la réaction de cyclisation *mode opératoire C*.
*RMN ¹H (300 MHz, CDCl₃:CD₃OD*/*90:10)* 3,09 (t, J=6,9 Hz, 2H); 3,82 (s, 3H); 4,2 (t, J=6,9 Hz, 2H); 6,79 (dd, J=2,4 et 8,8 Hz, 1H); 6,91 (d, J=2,4 Hz, 1H) ; 7,10 (s,1H); 7,23 (d, J=8,8 Hz, 1H); 7,50 (s,1H).
*SM (m*/*z)* 239 (100); 196; 168; 140.

### Exemple 4 : 8-méthoxy-3-méthyl-1-phényl-5,6-dihydroimidazo[5,1-a]-β-carboline

On procède comme dans l'exemple 1, en utilisant comme substrat lors du *mode opératoire* A la 5-méthoxytryptamine et la N-acétyl-2-phénylglycine, l'amide obtenu est directement engagé dans la réaction de cyclisation mode opératoire C.
*RMN ¹H (300 MHz, CDCl₃)* 2,5 (s, 3H); 3,15 (t, J=6,9 Hz, 2H); 3,85 (s, 3H), 4,08 (t, J=6,9 Hz, 2H); 6,8 (dd, J=2,4 et 8,8 Hz, 1H); 6,94 (d, J=2,4 Hz, 1H); 7,15 (d, J=8,8 Hz, 1H); 7,36 (m, 1H); 7,46 (m, 2H); 7,74 (m, 2H); 8,30 (s, 1H).
*SM* (m/z) 329 (100); 286; 165; 143.

### Exemple 5 : 1-éthyl-8-méthoxy-3-méthyl-5,6-dihydroimidazo[5,1-a]-β-carboline

On procède comme dans l'exemple 1, en utilisant comme substrat lors du *mode* opératoire A la 5-méthoxytryptamine et la N-acétyl-2-éthylglycine, l'amide obtenu est directement engagé dans la réaction de cyclisation *mode opératoire* C.
*RMN ¹H (300 MHz, CDCl₃)* 1,30 (t, 7Hz, 3H) ; 2,43 (s, 3H); 2,80(q, 7Hz, 2H); 3,11 (t, J=7 Hz, 2H); 3,88 (s, 3H), 4,06 (t, J=7 Hz, 2H); 6,83 (dd, J=2,4 et 8,7 Hz, 1H); 6,97 (d, J=2,4 Hz, 1H); 7,27 (d, J=8,7 Hz, 1H); 8,71 (s large, 1H).
*SM* (m/z) 281 (100); 266; 250; 233.

### Exemple 6 : 8-méthoxy-3-isopropyl-5,6-dihydroimidazo[5,1-a]-β-carboline

On procède comme dans l'exemple 1, en utilisant comme substrat lors du *mode opératoire A* la 5-méthoxytryptamine et la N-isobutyrylglycine, l'amide obtenu est directement engagé dans la réaction de cyclisation *mode opératoire* C.
*RMN ¹H* (*300 MHz, CDCl₃:CD₃OD*/*90:10)* 1,33 (d, J=6,8 Hz, 6H); 3,04 (m, 4H); 3,86 (s, 3H); 4,04 (t, J=6,8 Hz, 2H); 6,79 (d, J=8,76 Hz, 1H); 6,95 (s,1H); 6,98 (s,1H); 7,22 (d, J=8,76 Hz, 1H); 10,30 (s,1H).
*SM (m*/*z)* 281 (100); 266; 196; 133.

### Exemple 7 : 8-méthoxy-3-propyl-5,6-dihydroimidazo[5,1-a]-β-carboline

On procède comme dans l'exemple 1, en utilisant comme substrat lors du *mode opératoire A* la 5-méthoxytryptamine et la N-butyrylglycine, l'amide obtenu est directement engagé dans la réaction de cyclisation *mode opératoire* C.

*RMN ¹H (300 MHz, CDCl₃)* 1,00 (t, 7,5Hz, 3H); 1,76 (m, 2H); 2,70(t, 7,5Hz, 2H); 3,08 (t, J=7,5 Hz, 2H); 3,87 (s, 3H), 3,97 (t, J=7,5 Hz, 2H); 6,81 (dd, J=2,7 et 8,7 Hz, 1H); 6,88 (d, J=2,7 Hz, 1H)); 7,08 (s, 1H); 7,28 (d, J=8,7 Hz, 1H); 10,38 (s large, 1H).
*SM (m*/*z)* 281 M+.; 269; 252(100); 209.

### Exemple 8 : 8-méthoxy-11-méthyl-3-propyl-5,6-dihydroimidazo[5,1-a]-β-carboline méthane sulfonate

On procède comme dans l'exemple 1, en utilisant comme substrat lors du *mode opératoire A* la 5-méthoxytryptamine et la N-butyrylglycine.
*RMN ¹N(300 MHz, CDCl₃:CD₃OD*/*90:10)* 1,06 (t, 7,5Hz, 3H); 1,83 (m, 2H); 2,75(t, 7,5Hz, 2H); 3,08 (t, J=6,8 Hz, 2H); 3,8 (s, 3H), 3,88 (s, 3H), 4,06 (t, J=6,8 Hz, 2H); 6,86 (dd, J=2,4 et 8,7 Hz, 1H); 6,96 (d, J=2,4 Hz, 1H) ); 7,15 (s, 1H); 7,18 (d, J=8,7 Hz, 1H).
*SM (m*/*z)* 295 (100); 266; 223; 133.

### Exemple 9 : 8-méthoxy-3,11-diméthyl-5,6-dihydroimidazo[5,1a]-β-carboline méthane sulfonate

On procède comme dans l'exemple 1, en utilisant comme substrat lors du mode *opératoire* A la 5-méthoxytryptamine et la N-acétylglycine
*RMN ¹H (300 MHz, CDCl₃:CD₃OD*/*90:10)* 2,44 (s, 3H) ; ; 3,05 (t, J=6,9 Hz, 2H); 3,77 (s, 3H), 3,85 (s, 3H), 3,99 (t, J=6,9 Hz, 2H); 6,86 (dd, J=2,4 et 8,7 Hz, 1H); 6,93 (d, J=2,4 Hz, 1H)) ; 7,15 (d, J=8,7 Hz, 1H).
*SM (m*/*z)* 267 (100); 251; 235; 224.

### Exemple 10 : 8-hydroxy-3,11-diméthyl-5,6-dihydroimidazo[5,1-a]-β-carboline méthane sulfonate

On dissout la 8-méthoxy-3,11-diméthyl-5,6-dihydroimidazo[5,1-a]-β-carboline (exemple 9, m=300mg, 1mmol) dans du dichlorométhane anhydre à - 78°C. On ajoute ensuite du BBr₃ (V=8ml) et on laisse remonter à température ordinaire pendant 12 heures sous agitation et sous atmosphère d'azote. On ajoute d'une solution (2M) NaHCO₃ (15 ml) après décantation le produit précipite dans le dichlorométhane, on récupère le produit attendu (m=150 mg, 0,6mmol, Rdt =50%).
*RMN 1H (300 MHz, CDCl₃:CD₃OD*/*90:10)* 2,45 (s, 3H) ; ; 3,05 (t, J=6,7 Hz, 2H), 3,84 (s, 3H), 4,06 (t, J=6,7 Hz, 2H); 6,80 (d, J= 8,7 Hz, 1H); 6,91 (s, 1H)) ; 7,15 (d, J=8,7 Hz, 1H).
*SM* (*m*/*z*) 253 (100); 224; 211; 126.

### Exemple 11 : 8-hydroxy-3-méthyl-5,6-dihydroimidazo[5,1-a]-β-carboline méthane sulfonate

On dissout la 8-méthoxy-3-méthyl-5,6-dihydroimidazo[5,1-a]-β-carboline (exemple 2, m=350mg, 1,4mmol) dans du dichlorométhane anhydre à - 78°C. On ajoute ensuite du BBr₃ (V=8ml) et on laisse remonter à température ordinaire pendant 12 heures sous agitation et sous atmosphère d'azote. On ajoute d'une solution (2M) NaHCO₃ (15 ml) après décantation le produit précipite dans le dichlorométhane on récupère le produit attendu (m=200 mg, 0,83mmol, Rdt =59%)
*RMN ¹H (300 MHz, CDCl₃:CD₃OD*/*90:10)* 2,67 (s, 3H) ; ; 3,19 (t, J=7,2 Hz, 2H); 4,21 (t, J=7,2 Hz, 2H); 6,78 (dd, J=2,4 et 8,7 Hz, 1H); 6,88 (d, J=2,4 Hz, 1H)); 7,23 (d, J=8,7 Hz, 1H).

### Exemple 12 : 3-méthyl-5,6-dihydroimidazo[5,1-a]-β-carboline méthane sulfonate

On procède comme dans l'exemple 1, en utilisant comme substrat lors du mode *opératoire A* la tryptamine et la N-acétylglycine, l'amide obtenu est directement engagé dans la réaction de cyclisation mode *opératoire* C.
*RMN ¹H (300 MHz, CDCl₃)* 2,53 (s, 3H);; 3,08 (t, J=6,8 Hz, 2H), 3,99 (t, J=6,8 Hz, 2H); 7,04 (m, 2H), 7,16 (m, 2H); 7,36 (s, 1H); 7,44 (d, 1H)
*SM (m*/*z)* 223 (100); 208; 181; 154.

### Exemple 13 : 8-chloro-3-méthyl-5,6-dihydroimidazo[5,1-a]-β-carboline méthane sulfonate

On procède comme dans l'exemple 1, en utilisant comme substrat lors du *mode opératoire A* la 5-chlorotryptamine et la N-acétylglycine, l'amide obtenu est directement engagé dans la réaction de cyclisation mode *opératoire* C.

*RMN ¹H (300 MHz, CDCl₃:CD₃OD*/*90:10)* 2,72 (s, 3H); 2,81 (s, 3H); 3,16 (t, J=6,9 Hz, 2H); 4,13 (t, J=6,9 Hz, 2H); 7,12 (dd, J=2 et 8,7 Hz, 1H); 7,31 (d, J=8,7 Hz, 1H); 7,44 (d, J=2 Hz, 1H) ); 7,52 (s, 1H)
*SM (m*/*z)* 257 (100); 242; 221; 215.

### Exemple 14 : 8-méthoxy-3-phényl-5,6-dihydroimidazo[5,1-a]-β-carboline

On procède comme dans l'exemple 1, en utilisant comme substrat lors du *mode opératoire* A la 5-méthoxytryptamine et l'acide hippurique, l'amide obtenu est directement engagé dans la réaction de cyclisation mode *opératoire* C.
*RMN ¹H* (*300 MHz,* CD₃COCD₃) 2,87 (s, 3H); 3,16 (t, J=6,8 Hz, 2H) ; 3,82 (s, 3H), 4,45 (t, J=6,8Hz, 2H); 6,77 (dd, J=2,4 et 9,6 Hz, 1H) ; 7,05 (d, J=2,4 Hz, 1H); 7,30 (s,1H); 7,31 (d, J=9,6 Hz, 1H); 7,52 (m, 3H); 7,75 (m, 2H); 8,02 (s, 1H).
*SM* (*m*/*z*) 315 (100); 272; 211; 168

### Exemple 15 : 11-éthyl-3-méthyl-5,6-dihydroimidazo[5,1-a]-β-carboline méthane sulfonate

On procède comme dans l'exemple 1, en utilisant comme substrat lors du *mode opératoire* A la tryptamine et la N-acétylglycine. En utilisant comme agent d'alkylation pour le *mode opératoire B* le bromure d'éthyle
*RMN ¹H (300 MHz, CD₃OD)* 1,33 (t, 7,1 Hz, 3H); 2,76(s, 3H); 2,78 (s, 3H); 3,25 (t, J=6,9 Hz, 2H), 4,24 (m, 4H); 7,12 (t, 1H); 7,3 (m, 2H) ); 7,49 (d+ls, 2H).

### Exemple 16 : 8-chloro-3,11-diméthyl-5,6-dihydroimidazo[5,1-a]-β-carboline méthane sulfonate

On procède comme dans l'exemple 1, en utilisant comme substrat lors du mode *opératoire* A la 5-chlorotryptamine et la N-acétylglycine.
*RMN ¹H (300 MHz, CDCl₃)* 2,60 (s, 3H); 2,70 (s, 3H); 3,16 (t, J=6,8 Hz, 2H) ; 4,20 (t, J=6,8 Hz, 2H) ; 7,12 (d, J=8,8 Hz, 1H) ; 7,25 (d, J=8,8 Hz, 1H); 7,50 (s, 1H)); 7,6 (s, 1H)
***SM (m*/*z)*** 271 (100); 235; 193; 167.

### Exemple 17 : 8-chloro-3-méthyl-11-ethyl-5,6-dihydroimidazo[5,1-a]-β-carboline méthane sulfonate

On procède comme dans l'exemple 1, en utilisant comme substrat lors du *mode opératoire A* la 5-chlorotryptamine et la N-acétylglycine. En utilisant comme agent d'alkylation pour le mode *opératoire* B le bromure d'éthyle.
*RMN ¹H (300 MHz, CDCl₃)* 1,39 (t, J=4,8 Hz, 3H); 2,70 (s, 3H); 2,85 (s, 3H); 3,2 (t, J=7 Hz, 2H); 4,3 (m, 4H); 7,2 (dd, J=2,4 et 8,8 Hz, 1H); 7,35 (d, J=8,8 Hz, 1H); 7,53 (d, J=2,4 Hz, 1H)); 7,6 (s, 1H)
*SM (m*/*z)* 285 (100); 270; 249; 180

### Exemple 18 : 3,8-diméthyl-5,6-dihydroimidazo[5,1-a]-β-carboline méthane sulfonate

On procède comme dans l'exemple 1, en utilisant comme substrat lors du *mode opératoire A* la 5-méthyltryptamine et la N-acétylglycine, l'amide obtenu est directement engagé dans la réaction de cyclisation mode *opératoire* C.
*RMN ¹H (300 MHz, CDCl₃:CD₃OD*/*90:10)* 2,43 (s, 3H) ; 2,61 (s, 3H) ; 2,85 (s, 3H); 3,14 (t, J=7 Hz, 2H); 4,08 (t, J=7 Hz, 2H); 7,05 (d, 8,6 Hz, 1H); 7,28 (s, 1H), 7,32 (d, J=8,6 Hz, 1H); 7,51 (s, 1H) )
*SM* (m/z) 237 (50); 129; 73; 55 (100).

### Exemple 19 : 3,8,11-triméthyl-5,6-dihydroimidazo[5,1-a]-β-carboline méthane sulfonate

On procède comme dans l'exemple 1, en utilisant comme substrat lors du *mode opératoire A* la 5-méthyltryptamine et la N-acétylglycine.
*RMN ¹H (CDCl₃:CD₃OD*/*90:10)* 2,40 (s, 3H); 2,70 (s, 3H); 2,75 (s, 3H); 3,2 (t, J=6,9 Hz, 2H);3,75 (s, 3H); 4,18 (t, J=6,9 Hz, 2H); 7,07 (d, J=8,5 Hz, 1H), 7,21 (d, J=8,5 Hz, 1H); 7,27 (s, 1H); 7,50 (s, 1H)
*SM* (*m*/*z*) 251 (100); 235; 203.

### Exemple 20 : 11-éthyle-3,8-diméthyl-5,6-dihydroimidazo[5,1-a]-β-carboline méthane sulfonate

On procède comme dans l'exemple 1, en utilisant comme substrat lors du *mode opératoire A* la 5-méthyltryptamine et la N-acétylglycine. En utilisant comme agent d'alkylation pour le mode *opératoire B* le bromure d'éthyle.
*RMN ¹H* (*300 MHz, CDCl₃:CD₃OD*/*90:10)* 1,32 (t, J=7,2 Hz, 3H); 2,40 (s, 3H); 2;72 (s, 3H); 2,78 (s, 3H); 3,21 (t, J=6,9 Hz, 2H); 4,19 (m, 4H); 7,08 (d, J= 8,4 Hz, 1H); 7,20 (d, J=8,4 Hz, 1H); 7,29 (s, 1H) ); 7,45 (s, 1H)
*SM (m*/*z)* 265 (100); 250; 236; 223.

### Exemple 21 : 8-fluoro-3-méthyl-5,6-dihydroimidazo[5,1-a]-β-carboline méthane sulfonate

On procède comme dans l'exemple 1, en utilisant comme substrat lors mode *opératoire* A la 5-fluorotryptamine et la N-acétylglycine, l'amide obtenu est directement engagé dans la réaction de cyclisation *mode opératoire* C.
*RMN ¹H (300 MHz, CDCl₃:CD₃OD*/*90:10)* 2,60 (s, 3H); 2,75 (s, 3H); 3,09 (t, J=7 Hz, 2H); 4,10 (t, J=7 Hz, 2H); 6,87 (dt, J=2,4 et 9Hz, 1H), 7,02 (dd, J= 2,4 et 9 Hz, 1H); 7,26 (dd, J=3 et 9 Hz, 1H); 7,50 (s, 1H).
*SM (m*/*z)* : 241 (100); 226; 199; 172.

### Exemple 22 : 8-bromo-3-méthyl-5,6-dihydroimidazo[5,1-a]-β-carboline méthane sulfonate

On procède comme dans l'exemple 1, en utilisant comme substrat lors du mode *opératoire A* la 5-bromotryptamine et la N-acétylglycine, l'amide obtenu est directement engagé dans la réaction de cyclisation *mode opératoire* C.
*RMN ¹H(300 MHz*, *CDCl₃:CD₃OD*/*90:10)* 2,68 (s, 3H); 2,86 (s, 3H); 3,19 (t, J=7,2 Hz, 2H); 4,20 (t, J=7,2 Hz, 2H); 7,28 (m, 2H); 7,34 (s, 1H); 7,58 (s, 1H) ; 7,61 (s, 1H) .
*SM* (m/z) 301/302 (100); 286; 259; 234.

### Exemple 23 : 8-fluoro-3,11 diméthyl-5,6-dihydroimidazo[5,1-a]-β-carboline méthane sulfonate

On procède comme dans l'exemple 1, en utilisant comme substrat lors du *mode opératoire* A la 5-fluorotryptamine et la N-acétylglycine.
*RMN ¹H (300 MHz, CDCl₃:CD₃OD*/*90:10)* 2,78 (s, 3H); 2,83 (s, 3H) ; 3,25 (t, J=6,9 Hz, 2H); 4,31 (t, J=6,9 Hz, 2H); 7,07 (dt, J=2,4 et 9Hz, 1H), 7,22 (dd, J= 2,4 et 9 Hz, 1H); 7,32 (dd, J=3 et 9 Hz, 1H); 7,66 (s, 1H).
*SM (m*/*z)* 255 (100); 213; 185; 128.

### Exemple 24 : 8-fluoro-11-éthyl-3méthyl-5,6-dihydroimidazo[5,1-a]-β-carboline méthane sulfonate

On procède comme dans l'exemple 1, en utilisant comme substrat lors du *mode opératoire A* la 5-fluorotryptamine et la N-acétylglycine. En utilisant comme agent d'alkylation pour le *mode opératoire B* le bromure d'éthyle.
*RMIV ¹H (CDCl₃:CD₃OD*/*90:10)* 1,32 (t, J=7,2 Hz, 3H); 2,70 (s, 3H); 2,80 (s, 3H); 3,16 (t, J=6,9 Hz, 2H); 4,2 (m, 4H); 6,97 (dt, J=2,4 et 9Hz, 1H) , 7,13 (dd, J= 2,4 et 9 Hz, 1H) ; 7,23 (dd, J=4 et 9 Hz, 1H) ; 7,65 (s, 1H) .
*SM (m*/*z)* 269 (100); 254; 227; 199.

### Exemple 25 : 8-bromo-11-éthyl-3méthyl-5,6-dihydroimidazo[5,1-a]-β-carboline méthane sulfonate

On procède comme dans l'exemple 1, en utilisant comme substrat lors du *mode opératoire A* la 5-bromotryptamine et la N-acétylglycine. En utilisant comme agent d'alkylation pour le mode *opératoire* B le bromure d'éthyle.
*RMN ¹H (300 MHz, CDCl₃:CD₃OD*/*90:10)* 1,32 (t, J=7 Hz, 3H); 2,70 (s, 3H); 2,75 (s, 3H); 3,18 (t, J=7 Hz, 2H); 3,59 (s, 3H); 4,20 (m, 4H); 7,19 (d, J=8,7 Hz, 1H), 7,30 (dd, J=1,8 et 8,7 Hz, 1H); 7,53 (s, 1H); 7,63 (d, J=1,8Hz , 1H).
*SM (m*/*z)* 329/330 (100); 316; 300; 273.

Les dérivés dihydroimidazo[5,1-a]-β-carboline selon l'invention, en particulier ceux sous forme de méthyl-sulfonates (solubles) ont été testés chez le poussin et certains composés actifs chez le poussin ont été administrés en doses uniques chez (6) chiens Beagles des deux sexes pour une étude polysomnographique de durée 4 heures au moins.

### Protocole expérimental chez le poussin

Les poussins de souche JA 657 provenant du Couvoir Gauguet, 44 Le Pin, sont habitués pendant 6 jours au moins à un programme d'éclairement alterné de 12h de jour et 12h de nuit à température régulée à 25 +/- 2°C. Ils sont nourris ad libitum et sont mis en essai en vivarium par groupes de 3 individus de poids moyen 100 +/-10 g le jour de l'essai. A cet âge, il n'existe pas dans cette espèce de barrière méningée (hémato-encéphalique) efficace. Les produits de test sont injectés à 3 doses (1, 3 et 10 mg/kg) par voie IM en solution ou en suspension aqueuse (1 goutte de Tween 80 par ml), chacun à deux lots de 3 poussins qui sont observés pendant 90 minutes. Pour chaque série de test (18 vivariums) il existe au moins un lot de poussins témoins négatifs recevant le même volume (0.2 ml IM) d'eau pour préparations injectables.

Pendant la période de 90 minutes d'observation, les poussins sont stimulés fortement toutes les 15 minutes par la présentation d'une mangeoire pleine, et à chaque période de 5 minutes est attribuée une note correspondant à l'état de vigilance sur la période parmi les 5 états suivants :

Mobile, couché vigile, assoupi, sommeil, sommeil debout (sleep-like state). Les paramètres étudiés sont le délai d'assoupissement (TA) écoulé entre l'injection et le premier stade de sommeil, la durée du premier sommeil (TS) et le temps total de sédation sur la période (Tsed) exprimé en minutes et en % de la période (Sed).

Pour comparer les résultats d'essais réalisés à différentes dates, l'allongement de la durée du premier sommeil TS par rapport aux lots témoins de l'essai a été rapporté.

### Résultats

Chez le poussin de cet âge hors essai, la durée d'un cycle veille-sommeil est de 20 à 30 minutes pendant la journée. Il apparaît donc, dès la dose d'1 mg que 11 composés (sur 20) au moins induisent une diminution très forte de l'activité locomotrice attestée par une durée du premier sommeil TS supérieur à 20 minutes.

Le nombre des produits de test dans ce cas aux doses plus élevées passe à 18/20 et à 20/20 à 3 et à 10 mg/kg.

Il existe une relation positive dose-effet nette pour la plupart des composés testés, avec une réduction du délai d'assoupissement lorsque la dose augmente.

Sur 90 minutes , l'écart du temps de sédation avec celui observé avec le placebo est supérieur à 52 minutes pour 2 composés dès la dose d'1 mg/kg, pour 4 composés à 3 mg/kg et pour 12 composés à 10 mg/kg.

**Tableau II (Dose : 1 mg/kg)**

| COMPOSES | TA | TS | Tsed | Sed | TS (minutes) |
|---|---|---|---|---|---|
| | Minutes | Minutes | Minutes | % | Ecart Placebo |
| Exemple 1 | 7 | 56,5 | 64,5 | 71,7 | 56 |
| Exemple 2* | 16 | 3 | 25 | 27,8 | 3 |
| Exemple 3* | 10 | 40 | 66,51 | 73,9 | 36 |
| Exemple 4* | 16,5 | 38 | 53,01 | 58,9 | 34 |
| Exemple 5* | 20,5 | 33 | 52,47 | 58,3 | 29 |
| Exemple 6* | 11 | 56,5 | 65,97 | 73,3 | 52,5 |
| Exemple 7* | 16,5 | 16 | 46 | 41,4 | 16 |
| Exemple 8 | 16 | 21,5 | 38 | 42,2 | 21 |
| Exemple 9 | 22 | 3 | 24,5 | 27,2 | 2,8 |
| Exemple 10 | 15 | 5 | 38 | 42,2 | 4,7 |
| Exemple 11 | 40 | 0 | 26,5 | 29,4 | -0,3 |
| Exemple 12 | 10 | 39,5 | 46,5 | 51,7 | 39 |
| Exemple 13 | 16,5 | 33,5 | 52 | 57,8 | 33 |
| Exemple 14* | 7 | 35,5 | 62,865 | 63,5 | 32 |
| Exemple 15 | 12,5 | 12 | 21,5 | 23,9 | 21 |
| Exemple 16 | 11,5 | 15 | 37 | 41 | 14,7 |
| Exemple 17 | 12 | 12 | 37 | 41,1 | 11,7 |
| Exemple 18 | 5 | 32,5 | 62,5 | 69,4 | 24 |
| Exemple 19 | 4 | 17,5 | 44,5 | 49,4 | 11 |
| Exemple 20 | 2 | 65,5 | 72 | 80 | 57 |

| | | | | | |
|---|---|---|---|---|---|
| * sous forme de base libre | | | | | |

**Tableau III (Dose : 3mg/Kg)**

| COMPOSES | TA | TS | Tsed | Sed | TS (minutes) |
|---|---|---|---|---|---|
| | Minutes | Minutes | Minutes | % | Ecart Placebo |
| Exemple 1 | 4,5 | 78,5 | 78,5 | 87,2 | 78 |
| Exemple 2* | 8,5 | 41 | 62,5 | 69,4 | 41 |
| Exemple 3* | 7 | 66,5 | 71,0 | 78,9 | 62,5 |
| Exemple 4* | 14 | 22,5 | 65,5 | 72,8 | 18,5 |
| Exemple 5* | 14,5 | 53 | 69,0 | 76,7 | 49 |
| Exemple 6* | 12,5 | 72,5 | 72,5 | 80,6 | 68,5 |
| Exemple 7* | 16,5 | 27 | 45,5 | 41 | 27 |
| Exemple 8 | 8,5 | 33 | 54 | 60 | 32,7 |
| Exemple 9 | 17,5 | 13,5 | 26,5 | 29,4 | 13 |
| Exemple 10 | 30 | 1 | 11 | 12,2 | 0,7 |
| Exemple 11 | 15 | 7 | 34,5 | 38,3 | 6,7 |
| Exemple 12 | 3,5 | 42 | 57,5 | 63,9 | 41,7 |
| Exemple 13 | 9,5 | 73 | 73 | 81,1 | 73 |
| Exemple 14* | 15 | 34 | 61,2 | 68 | 31,5 |
| Exemple 15 | 7 | 29,5 | 40,5 | 45 | 29 |
| Exemple 16 | 13,5 | 20,5 | 30,5 | 33,9 | 20 |
| Exemple 17 | 7,5 | 23,5 | 30 | 33,3 | 23 |
| Exemple 18 | 4,5 | 30,5 | 57,5 | 63,9 | 22,5 |
| Exemple 19 | 3,5 | 46,5 | 56,5 | 62,8 | 38,5 |
| Exemple 20 | 1,5 | 51,5 | 71,5 | 79,4 | 43,5 |

| | | | | | |
|---|---|---|---|---|---|
| * Sous forme de base libre | | | | | |

**Tableau IV (Dose: 10 mg/kg)**

| COMPOSES | TA | TS | Tsed | Sed | TS (minutes) |
|---|---|---|---|---|---|
| | Minutes | Minutes | Minutes | % | Ecart Placebo |
| Exemple 1 | 1 | 86 | 87 | 96,7 | 85,7 |
| Exemple 2* | 7 | 83 | 83 | 92,2 | 83 |
| Exemple 3* | 6,5 | 77,5 | 78,0 | 86,7 | 73,5 |
| Exemple 4* | 8,5 | 81,5 | 81,5 | 90,6 | 76,5 |
| Exemple 5* | 13,5 | 40,5 | 56,5 | 62,8 | 36,5 |
| Exemple 6* | 12,5 | 72,5 | 74,0 | 82,2 | 68,5 |
| Exemple 7* | 12,5 | 38,5 | 53,5 | 48,2 | 38,5 |
| Exemple 8 | 5,5 | 41 | 68,5 | 76,1 | 40,7 |
| Exemple 9 | 16,5 | 15,5 | 38,5 | 42,8 | 15 |
| Exemple 10 | 11,5 | 12,5 | 27 | 30 | 12 |
| Exemple 11 | 6 | 9,5 | 18,5 | 20,6 | 9 |
| Exemple 12 | 1 | 89 | 89 | 98,9 | 88,7 |
| Exemple 13 | 4 | 86 | 86 | 95,6 | 85,7 |
| Exemple 14* | 7 | 28 | 63 | 70 | 25,5 |
| Exemple 15 | 1 | 70 | 72 | 80 | 69,7 |
| Exemple 16 | 7,5 | 31 | 52,5 | 58,3 | 52 |
| Exemple 17 | 5 | 43,5 | 60,5 | 67,2 | 43 |
| Exemple 18 | 7,5 | 76 | 81,5 | 90,6 | 68 |
| Exemple 19 | 3,5 | 60 | 67,5 | 75,0 | 52 |
| Exemple 20 | 1,5 | 87,5 | 87,5 | 97,2 | 81,5 |

| | | | | | |
|---|---|---|---|---|---|
| * Sous forme de base libre | | | | | |

### Protocole d'expérimentation chez le chien

Les essais polysomnographiques sont réalises pour chacun des 3 produits de la série, sur 6 chiens des 2 sexes, adultes provenant d'élevage agréé HARLAN à 03 Gannat, avec des électrodes en inox implantées chirurgicalement pour la durée de l'essai au contact des os frontaux au travers des sinus, en regard des aires motrices de l'encéphale. La méthode adaptée de Nishino et coll. par Tafani, Valin et coll. a été décrite par ailleurs. Elle comprend l'enregistrement de 2 voies pour les mouvements oculaires, une voie musculaire (électrode concentrique dans les muscles de la nuque) et 2 voies électroencéphalographiques(EEG). L'enregistrement EEG numérisé est réalisé sur un polygraphe Nicollet Schwarzer ou similaire, (DELTAMED Cohérence 2 et 3, MEI Galileo NT) pendant 4 heures après administration d'une gélule 00 contenant 0, 16 ou 48 mg de produit de test( soit en moyenne 0 1 et 3 mg/kg de poids vif).

Chaque chien est enregistré 4 heures pendant 2 fois 2 jours consécutifs soit le matin, soit l'après-midi, après une semaine d'habituation à la cage d'enregistrement, 1 m2, 80 cm de haut en inox, type SHOR-LINE. Pour chaque chien l'enregistrement commence toujours à la même heure. Un repas est distribué 30 minutes avant le début de l'enregistrement.

Les tracés polysomnographiques permettent de distinguer 4 stades au moins pour chaque époque de 30 secondes consécutives la veille, la somnolence, le sommeil lent et le sommeil paradoxal. La latence d'apparition de chacun des premiers épisodes non vigiles et la durée de chacun des stades par période de 2 heures sont obtenus pour chaque chien. La comparaison des moyennes permet d'étudier l'effet des produits d'essai sur la microstructure du sommeil.

### Résultats :

Latences : Tableaux V à VII :

**Tableau V**

| Dose | Somnolence minutes | Sommeil à ondes lentes (SWS) minutes | Sommeil paradoxal (REM) minutes |
|---|---|---|---|
| Placebo 1 + 2 | 70 +/- 6 | 98 +/- 20 | 137 +/- 4 |
| Exemple 2 (1 mg) | 40 +/- 14.7 | 44 +/- 16.3 | 114 +/- 53 |
| Exemple 2 (3 mg) | 37 +/- 6.7 | 45 +/- 5.4 | 63 +/- 21.1 |

**Tableau VI**

| Dose | Somnolence minutes | Sommeil à ondes lentes (SWS) minutes | Sommeil paradoxal (REM) minutes |
|---|---|---|---|
| Placebo 1 | 69 +/- 58 | 79 +/- 60 | 100 +/- 62 |
| Exemple 13 (1 mg) | 25 +/- 11.6 | 32 +/- 11.5 | 63 +/- 28.2 |
| Placebo 2 | 40 +/- 13.1 | 50 +/- 23.8 | 62 +/- 22.4 |
| Exemple 13 (3 mg) | 27 +/- 9.1 | 35 +/- 11 | 56 +/- 19.5 |

**Tableau VII**

| Dose | Somnolence minutes | Sommeil à ondes lentes (SWS) minutes | Sommeil paradoxal (REM) Minutes |
|---|---|---|---|
| Placebo 1 | 71 +/- 39.8 | 102 +/- 71.8 | 125 +/- 66.9 |
| Exemple 1 (1 mg) | 47 +/- 19.1 | 52 +/- 24.8 | 101 +/- 80.3 |
| Placebo 2 | 44 +/- 20.4 | 61 +/- 27.6 | 87 +/- 30.2 |
| Exemple 1 (3 mg) | 21 +/- 7.1 | 27 +/- 8.0 | 50 +/- 22.2 |

On observe avec les trois produits d'essai une nette diminution de la latence de la première somnmolence, plus nette à la dose de 3 mg/kg pour le produit de l'exemple 1. La latence du premier épisode de sommeil lent (caractérisé par des fuseaux sur les tracés EEG) est divisée par deux avec l'ensemble des produits d'essai.

Les trois composés induisent un effet hypnagogique positif.

Temps passé à chaque stade de vigilance : Tableaux VIII à X

**Tableau VIII**

| Dose | Veille minutes | Somnolence minutes | SWS Minutes | REM minutes |
|---|---|---|---|---|
| Placebo 1 + 2 | 147 +/- 35.5 | 25 +/- 10.3 | 59 +/- 21.9 | 8.5 +/- 5.4 |
| Exemple 2 (1 mg) | 136 +/- 40.3 | 31 +/- 4.6 | 59 +/- 20.5 | 13.5 +/- 10.6 |
| Exemple 2 (3 mg) | 123 +/- 30.5 | 36 +/- 15.4 | 60 +/- 26.9 | 20 +/- 9.5 |

**Tableau IX**

| Dose | Veille minutes | Somnolence minutes | SWS minutes | REM Minutes |
|---|---|---|---|---|
| Placebo 1 | 156 +/- 38.5 | 26 +/- 11.3 | 39 +/- 23.9 | 19 +/- 11.4 |
| Exemple 13 (1 mg) | 135 +/- 44.3 | 24 +/- 2.6 | 57 +/- 26.5 | 21 +/- 13.6 |
| Placebo 2 | 140 +/- 27.9 | 32 +/- 7.3 | 47 +/- 19.9 | 21 +/- 10 |
| Exemple 13 (3 mg) | 122 +/- 36.5 | 31 +/- 7 | 61 +/- 24.9 | 26 +/- 13.5 |

**Tableau X**

| Dose | Veillle minutes | Somnolence minutes | SWS minutes | REM minutes |
|---|---|---|---|---|
| Placebo 1 | 154 +/- 35.2 | 30 +/- 16.0 | 45 +/- 19.4 | 11 +/- 8.5 |
| Exemple 1 (1 mg) | 139 +/- 69.1 | 27 +/- 10.4 | 51 +/- 42.3 | 23 +/- 19.1 |
| Placebo 2 | 134 +/- 36.5 | 35 +/- 21.4 | 50 +/- 34.8 | 20 +/- 10.8 |
| Exemple 1 (3 mg) | 106 +/- 34.7 | 40 +/- 10.9 | 65 +/- 30.2 | 29 +/- 13.7 |

On observe au cours des 4 heures d'enregistrement une tendance à la diminution du temps de veille en particulier à la dose de 3 mg/kg pour les trois composés. Cette diminution de la veille se fait essentiellement par augmentation des stades de sommeil lent et de sommeil paradoxal .

Les effets observés apparaissent plus nettement au cours des 2 premières heures, particulièrement à la dose d'1 mg/kg, ce qui peut correspondre à la cinétique d'élimination des produits.

Les trois composés présentent des propriétés hypnotiques.

A la différence des composés connus, il n'y a ni modification du pourcentage de somnolence ni de celui du sommeil paradoxal dans le temps non vigile.

Les dérivés imidazopyridoindoles selon l'invention présentent en commun une activité pharmacologique sur le système nerveux central de deux espèces animales au moins. Ils diminuent la vigilance, accélèrent l'apparition du sommeil (effet hypnagogique positif). Chez le poussin et chez le chien, il y a diminution de l'activité vigile. Dans cette dernière espèce il n'y a pas de modification de l'ultrastructure du sommeil. Ils sont donc utiles en tant que médicaments en particulier comme hypnotiques.

## Revendications

1. Composés de dihydroimidazo[5,1-a]-β-carboline de formule générale (I) 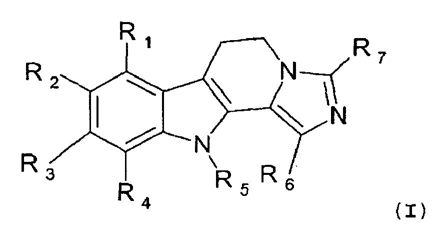 dans laquelle :
R₂ représente un atome d'hydrogène, d'halogène, un groupement alkyle (C₁-C₆) linéaire ou ramifié, hydroxy, alkoxy (C₁-C₆) linéaire ou ramifié, trihalogénoalkyle (C₁-C₆) linéaire ou ramifié ou trihalogénoalkoxy (C₁-C₆) linéaire ou ramifié,
R₁, R₃ et R₄ représentent un atome d'hydrogène,
R₆ et R₇, identiques ou différents, représentent indépendamment l'un de l'autre un atome d'hydrogène, un alkyle (C₁-C₆) linéaire ou ramifié ou un phényle, et
R₅ représente un atome d'hydrogène ou un alkyle (C₁-C₆) linéaire ou ramifié,
à l'exception toutefois des composés de formule générale I dans laquelle R₁ à R₆ représentent un atome d'hydrogène et R₇ représente un groupe CH₃ ou phényle,
ainsi que leurs sels d'addition à un acide pharmaceutiquement acceptable.

2. Composés selon la revendication 1, **caractérisés en ce que**:
R₂ représente un atome d'hydrogène, un atome d'halogène (fluor, chlore ou brome), un alkyle (C₁-C₆) linéaire ou ramifié), un hydroxy, un alkoxy (C₁-C₆) linéaire ou ramifié).

3. Composés selon l'une des revendications 1 et 2,
**caractérisés en ce qu'**ils sont choisis parmi les composés suivants :
3,11-diméthyl-5,6-dihydroimidazo[5,1-a]-β-carboline méthane sulfonate
8-méthoxy-3-méthyl-5,6-dihydroimidazo[5,1-a]-β-carboline méthane sulfonate
8-méthoxy-5,6-dihydroimidazo[5,1-a]-β-carboline
8-méthoxy-3-méthyl-1-phényl-5,6-dihydroimidazo[5,1-a]-β-carboline
1-éthyl-8-méthoxy-3-méthyl-5,6-dihydroimidazo[5,1-a]-β-carboline
8-méthoxy-3-isopropyl-5,6-dihydroimidazo[5,1-a]-β-carboline
8-méthoxy-3-propyl-5,6-dihydroimidazo[5,1-a]-β-carboline
8-méthoxy-11-méthyl-3-propyl-5,6-dihydroimidazo[5,1-a]-β-carboline méthane sulfonate
8-méthoxy-3,11-diméthyl-5,6-dihydroimidazo[5,1-a]-β-carboline méthane sulfonate
8-hydroxy-3,11-diméthyl-5,6-dihydroimidazo[5,1-a]-β-carboline méthane sulfonate
8-hydroxy-3-méthyl-5,6-dihydroimidazo[5,1-a]-β-carboline méthane sulfonate
8-chloro-3-méthyl-5,6-dihydroimidazo[5,1-a]-β-carboline méthane sulfonate
8-méthoxy-3-phényl-5,6-dihydroimidazo[5,1-a]-β-carboline
11-éthyl-3-méthyl-5,6-dihydroimidazo[5,1-a]-β-carboline méthane sulfonate
8-chloro-3,11-diméthyl-5,6-dihydroimidazo[5,1-a]-β-carboline méthane sulfonate
8-chloro-3-méthyl-11-ethyl-5,6-dihydroimidazo[5,1-a]-β-carboline méthane sulfonate 3,8-diméthyl-5,6-dihydroimidazo[5,1-a]-β-carboline méthane sulfonate
3,8,11-triméthyl-5,6-dihydroimidazo[5,1-a]-β-carboline méthane sulfonate
11-éthyle-3,8-diméthyl-5,6-dihydroimidazo[5,1-a]-β-carboline méthane sulfonate
8-fluoro-3-méthyl-5,6-dihydroimidazo[5,1-a]-β-carboline méthane sulfonate
8-bromo-3-méthyl-5,6-dihydroimidazo[5,1-a]-β-carboline méthane sulfonate
8-fluoro-3,11 diméthyl-5,6-dihydroimidazo[5,1-a]-β-carboline méthane sulfonate
8-fluoro-11-éthyl-3-méthyl-5,6-dihydroimidazo[5,1-a]-β-carboline méthane sulfonate
8-bromo-11-éthyl-3-méthyl-5,6-dihydroimidazo[5,1-a]-β-carboline méthane sulfonate

4. Procédé de préparation des composés de formule (I) selon l'une des revendications 1 à 3, **caractérisé en ce qu'**un composé de formule (II) : 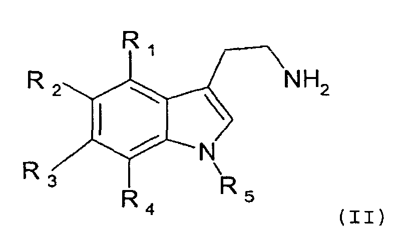 dans laquelle R₁, R₂, R₃, R₄ et R₅ ont la même signification que dans la formule (I), est mis à réagir, selon des conditions de synthèse de type couplage peptidique, avec un composé de formule (III) : 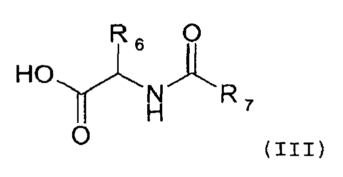 dans laquelle R₆ et R₇ sont tels que définis dans la formule (I), pour conduire au composé de formule (IV) : 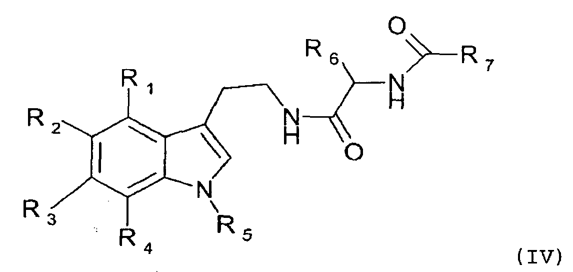 dans lequel R₁, R₂, R₃, R₄, R₅, R₆ et R₇ sont tels que définis précédemment, composé de formule (IV) que l'on traite en présence d'oxychlorure de phosphore dans un solvant tel que le toluène, pour conduire aux composés de formule (I), 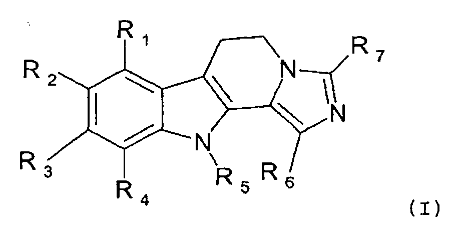 dans laquelle R₁, R₂, R₃, R₄, R₅, R₆ et R₇ sont tels que définis précédemment, les composés de formule générale (I) étant transformés, le cas échéant, en leurs sels d'addition à un acide pharmaceutiquement acceptable.

5. Compositions pharmaceutiques renfermant comme principe actif au moins un composé selon les revendications 1 à 3 ou un composé de formule générale I dans laquelle R₁ à R₆ représentent un atome d'hydrogène et R₇ représente un groupe CH₃ ou phényle, en combinaison avec un ou plusieurs excipients ou véhicules inertes, non toxiques, pharmaceutiquement acceptables.

6. Composé selon les revendications 1 à 3 ou composé de formule générale I dans laquelle R₁ à R₆ représentent un atome d'hydrogène et R₇ représente un groupe CH₃ ou phényle, à titre de médicaments.

7. Composé selon les revendications 1 à 3 ou composé de formule générale I dans laquelle R₁ à R₆ représentent un atome d'hydrogène et R₇ représente un groupe CH₃ ou phényle, à titre de médicaments hypnotiques.

## Claims

1. Compounds of dihydroimidazo[5,1-a]-β-carboline with general formula (I): 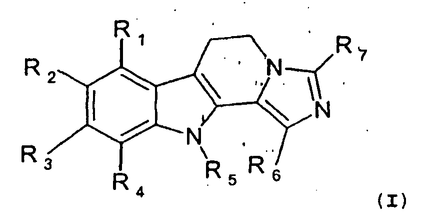 in which:
R₂ represents a hydrogen atom, a halogen atom, a linear or branched (C₁-C₆) alkyl group, a hydroxyl group, a linear or branched (C₁-C₆) alkoxy group, a linear or branched trihalogeno(C₁-C₆)alkyl group or a linear or branched trihalogeno(C₁-C₆)alkoxy group;
R₁, R₃ and R₄ represent a hydrogen atom;
R₆ and R₇, which may be identical or different, independently represent a hydrogen atom, a linear or branched (C₁-C₆) alkyl or a phenyl; and
R₅ represents a hydrogen atom or a linear or branched (C₁-C₆) alkyl;
with the exception, however, of compounds with general formula I in which R₁ to R₆ represent a hydrogen atom and R₇ represents a CH₃ group or a phenyl group;
and their addition salts with a pharmaceutically acceptable acid.

2. Compounds according to Claim 1, **characterized in that**:
R₂ represents a hydrogen atom, a halogen atom (fluorine, chlorine or bromine), a linear or branched (C₁-C₆) alkyl, a hydroxyl or a linear or branched (C₁-C₆) alkoxy.

3. Compounds according to either of Claims 1 and 2, **characterized in that** they are selected from the following compounds:
3,11-dimethyl-5,6-dihydroimidazo[5,1-a]-β-carboline methane sulfonate;
8-methoxy-3-methyl-5,6-dihydroimidazo[5,1-a]-β-carboline methane sulfonate;
8-methoxy-5,6-dihydroimidazo[5,1-a]-β-carboline;
8-methoxy-3-methyl-1-phenyl-5,6-dihydroimidazo[5,1-a]-β-carboline;
1-ethyl-8-methoxy-3-methyl-5,6-dihydroimidazo[5,1-a]-β-carboline;
8-methoxy-3-isopropyl-5,6-dihydroimidazo[5,1-a]-β-carboline;
8-methoxy-3-propyl-5,6-dihydroimidazo[5,1-a]-β-carboline;
8-methoxy-11-methyl-3-propyl-5,6-dihydroimidazo[5,1-a]-β-carboline methane sulfonate;
8-methoxy-3,11-dimethyl-5,6-dihydroimidazo[5,1-a]-β-carboline methane sulfonate;
8-hydroxy-3,11-dimethyl-5,6-dihydroimidazo[5,1-a]-β-carboline methane sulfonate;
8-hydroxy-3-methyl-5,6-dihydroimidazo[5,1-a]-β-carboline methane sulfonate;
8-chloro-3-methyl-5,6-dihydroimidazo[5,1-a]-β-carboline methane sulfonate;
8-methoxy-3-phenyl-5,6-dihydroimidazo[5,1-a]-β-carboline;
11-ethyl-3-methyl-5,6-dihydroimidazo[5,1-a]-β-carboline methane sulfonate;
8-chloro-3,11-dimethyl-5,6-dihydroimidazo[5,1-a]-β-carboline methane sulfonate;
8-chloro-3-methyl-11-ethyl-5,6-dihydroimidazo[5,1-a]-β-carboline methane sulfonate;
3,8-dimethyl-5,6-dihydroimidazo[5,1-a]-β-carboline methane sulfonate;
3,8,11-trimethyl-5,6-dihydroimidazo[5,1-a]-β-carboline methane sulfonate;
11-ethyl-3,8-dimethyl-5,6-dihydroimidazo[5,1-a]-β-carboline methane sulfonate;
8-fluoro-3-methyl-5,6-dihydroimidazo[5,1-a]-β-carboline methane sulfonate;
8-bromo-3-methyl-5,6-dihydroimidazo[5,1-a]-β-carboline methane sulfonate;
8-fluoro-3,11-dimethyl-5,6-dihydroimidazo[5,1-a]-β-carboline methane sulfonate;
8-fluoro-11-ethyl-3-methyl-5,6-dihydroimidazo[5,1-a]-β-carboline methane sulfonate;
8-bromo-11-ethyl-3-methyl-5,6-dihydroimidazo[5,1-a]-β-carboline methane sulfonate.

4. Method for preparing compounds with formula (I) according to one of claims 1 to 3, **characterized in that** a compound with formula (II): 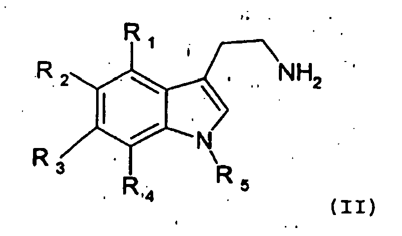 in which R₁, R₂, R₃, R₄ and R₅ have the same meaning as in formula (I), is reacted under peptide coupling synthesis conditions with a compound with formula (III): 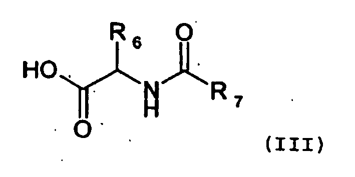 in which R₆ and R₇ are as defined in formula (I), to produce the compound with formula (IV): 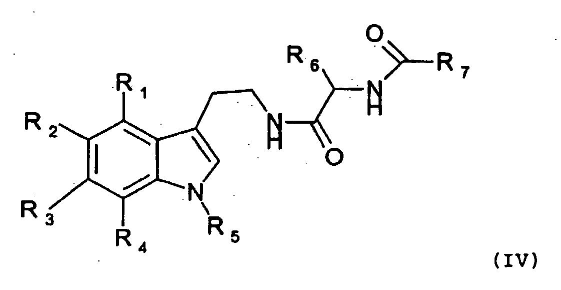 in which R₁, R₂, R₃, R₄, R₅, R₆ and R₇ are as defined above, the compound with formula (IV) being treated in the presence of phosphorus oxychloride in a solvent such as toluene to produce the compounds with formula (I) 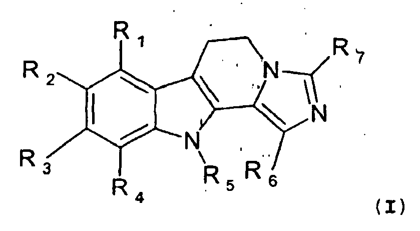 in which R₁, R₂, R₃, R₄, R₅, R₆ and R₇ are as defined above, the compounds with general formula (I) being transformed if appropriate into their addition salts with a pharmaceutically acceptable acid.

5. Pharmaceutical compositions comprising, as the active principle, at least one compound according to Claims 1 to 3 or one compound with general formula (I) in which R1 to R6 represent a hydrogen atom and R₇ represents a CH₃ group or a phenyl group, in combination with one or more nontoxic and pharmaceutically acceptable inert vehicles or excipients.

6. Compound according to Claims 1 to 3 or compound with general formula (I) in which R₁ to R₆ represent a hydrogen atom and R₇ represents a CH₃ group or a phenyl group, as medicaments.

7. Compound according to Claim 1 to 3 or compound with general formula (I) in which R₁ to R₆ represent a hydrogen atom and R₇ represents a CH₃ group or a phenyl group, as hypnotic medicaments.

## Patentansprüche

1. Verbindungen von Dihydroimidazo[5,1-a]-β-carbolin der allgemeinen Formel (I) 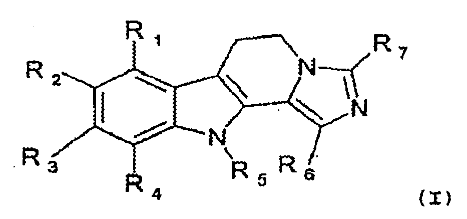 in der:
R₂ ein Wasserstoff-, Halogenatom, eine lineare oder verzweigte (C₁-C₆)-Alkyl-, Hydroxy-, lineare oder verzweigte (C₁-C₆)-Alkoxy-, lineare oder verzweigte (C₁-C₆)-Trihalogenalkyl- oder lineare oder verzweigte (C₁-C₆)-Trihalogenalkoxygruppe darstellt,
R₁, R₃ und R₄ ein Wasserstoffatom darstellen,
R₆ und R₇, identisch oder verschieden, unabhängig voneinander ein Wasserstoffatom, ein lineares oder verzweigtes (C₁-C₆)-Alkyl oder ein Phenyl darstellen und
R₅ ein Wasserstoffatom oder ein lineares oder verzweigtes (C₁-C₆)-Alkyl darstellen,
jedoch mit der Ausnahme von Verbindungen der allgemeinen Formel 1, in der R₁ bis R₆ ein Wasserstoffatom darstellen und R₇ eine CH₃- oder Phenylgruppe darstellt,
sowie deren Additionssalze mit einer pharmazeutisch annehmbaren Säure.

2. Verbindungen nach Anspruch 1, **dadurch gekennzeichnet, dass**:
R₂ ein Wasserstoffatom, ein Halogenatom (Fluor, Chlor oder Brom), ein lineares oder verzweigtes (C₁-C₆)-Alkyl, ein Hydroxy, ein lineares oder verzweigtes (C₁-C₆)-Alkoxy darstellt.

3. Verbindungen nach einem der Ansprüche 1 und 2, **dadurch gekennzeichnet, dass** sie aus den folgenden Verbindungen ausgewählt sind:
3,11-Dimethyl-5,6-dihydroimidazo[5,1-a]-β-carbolinmethansulfonat
8-Methoxy-3-methyl-5,6-dihydroimidazo[5,1-a]-β-carbolinmethansulfonat
8-Methoxy-5,6-dihydroimidazo[5,1-a]-β-carbolin
8-Methoxy-3-methyl-1-phenyl-5,6-dihydroimidazo[5,1-a]-β-carbolin
1-Ethyl-8-methoxy-3-methyl-5,6-dihydroimidazo[5,1-a]-β-carbolin
8-Methoxy-3-isopropyl-5,6-dihydroimidazo[5,1-a]-β-carbolin
8-Methoxy-3-propyl-5,6-dihydroimidazo[5,1-a]-β-carbolin
8-Methoxy-11-methyl-3-propyl-5,6-dihydroimidazo[5,1-a]-β-carbolinmethansulfonat
8-Methoxy-3,11-dimethyl-5,6-dihydroimidazo[5,1-a]-β-carbolinmethansulfonat
8-Hydroxy-3,11-dimethyl-5,6-dihydroimidazo[5,1-a]-β-carbolinmethansulfonat
8-Hydroxy-3-methyl-5,6-dihydroimidazo[5,1-a]-β-carbolinmethansulfonat
8-Chlor-3-methyl-5,6-dihydroimidazo[5,1-a]-β-carbolinmethansulfonat
8-Methoxy-3-phenyl-5,6-dihydroimidazo[5,1-a]-β-carbolin
11-Ethyl-3-methyl-5,6-dihydroimidazo[5,1-a]-β-carbolinmethansulfonat
8-Chlor-3,11-dimethyl-5,6-dihydroimidazo[5,1-a]-β-carbolinmethansulfonat
8-Chlor-3-methyl-11-ethyl-5,6-dihydroimidazo[5,1-a]-β-carbolinmethansulfonat
3,8-Dimethyl-5,6-dihydroimidazo[5,1-a]-β-carbolinmethansulfonat
3,8,11-Trimethyl-5,6-dihydroimidazo[5,1-a]-β-carbolinmethansulfonat
11-Ethyl-3,8-dimethyl-5,6-dihydroimidazo[5,1-a]-β-carbolinmethansulfonat
8-Fluor-3-methyl-5,6-dihydroimidazo[5,1-a]-β-carbolinmethansulfonat
8-Brom-3-methyl-5,6-dihydroimidazo[5,1-a]-β-carbolinmethansulfonat
8-Fluor-3,11-dimethyl-5,6-dihydroimidazo[5,1-a]-β-carbolinmethansulfonat
8-Fluor-11-ethyl-3-methyl-5,6-dihydroimidazo[5,1-a]-β-carbolinmethansulfonat
8-Brom-11-ethyl-3-methyl-5,6-dihydroimidazo[5, 1-a]-β-carbolinmethansulfonat.

4. Verfahren zur Herstellung von Verbindungen der Formel (I) nach einem der Ansprüche 1 bis 3, **dadurch gekennzeichnet, dass** eine Verbindung der Formel (II): 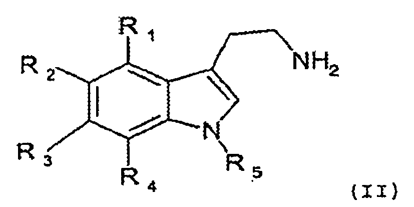 in der R₁, R₂, R₃, R₄ und R₅ die gleichen Bedeutungen wie in der Formel (I) aufweisen, gemäß Synthesebedingungen des Peptid-Kupplungstyps mit einer Verbindung der Formel (III): 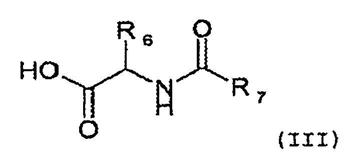 in der R₆ und R₇ wie in der Formel (I) definiert sind, umgesetzt wird, was zu einer Verbindung der Formel (IV): 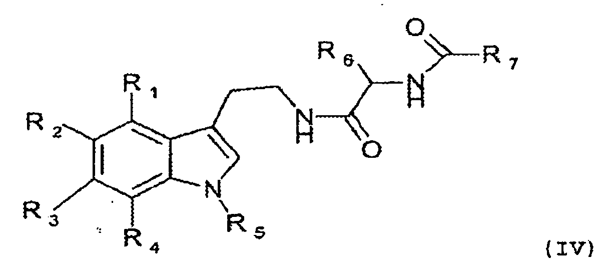 führt, in der R₁, R₂, R₃, R₄, R₅, R₆ und R₇ wie vorstehend definiert sind, man dann die Verbindung der Formel (IV) in Anwesenheit von Phosphoroxychlorid in einem Lösungsmittel wie Toluol behandelt, was zu Verbindungen der Formel (I) 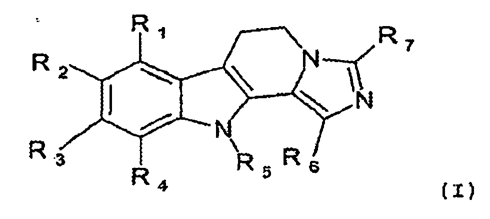 führt, in der R₁, R₂, R₃, R₄, R₅, R₆ und R₇ wie vorstehend definiert sind, wobei die Verbindungen der allgemeinen Formel (I) gegebenenfalls in ihre Additionssalze mit einer pharmazeutisch annehmbaren Säure überführt werden.

5. Pharmazeutische Zusammensetzungen, welche als Wirkstoff mindestens eine Verbindung nach den Ansprüchen 1 bis 3 oder eine Verbindung der allgemeinen Formel I, in der R₁ bis R₆ ein Wasserstoffatom darstellen und R₇ eine CH₃- oder Phenylgruppe darstellt, in Kombination mit einem oder mehreren inerten, nicht toxischen, pharmazeutisch annehmbaren Hilfsstoffen oder Trägern einschließt.

6. Verbindung nach den Ansprüchen 1 bis 3 oder Verbindung der allgemeinen Formel I, in der R₁ bis R₆ ein Wasserstoffatom darstellen und R₇ eine CH₃-Gruppe oder Phenyl darstellen, als Medikamente.

7. Verbindung nach den Ansprüchen 1 bis 3 oder Verbindungen der allgemeinen Formel 1, in der R₁ bis R₆ ein Wasserstoffatom darstellen und R₇ eine CH₃- oder Phenylgruppe darstellt, als hypnotische Medikamente.
